# FASCICULE DE BREVET EUROPEEN

(11) **EP 3 407 807 B1**
(45) Date de publication et mention de la délivrance du brevet: **07.10.2020**
(21) Numéro de dépôt: 17706531.5
(22) Date de dépôt: 13.01.2017
(51) Int. Cl.: A61B 17/16, A61B 90/00

(54) **DISPOSITIF DE COUPE FLEXIBLE ET CANULE**
FLEXIBLE UND KANÜLIERTE SCHNEIDVORRICHTUNG
FLEXIBLE AND CANNULATED CUTTING DEVICE

(30) Priorité: 18.01.2016 FR 1650369
(43) Date de publication de la demande: 05.12.2018
(73) Titulaire: Clariance, 62217 Beaurains (FR)
(72) Inventeur: LEROY, Jean Yves, 62870 Campagne Les Hesdin (FR); VIRGAUX, Nicolas, Paris 75018 (FR); BILLON, Adrien, 62217 Achicourt (FR); SCHULLER, Sébastien, 67200 Strasbourg (FR); VIART, Guy, 62128 Saint Leger (FR)
(74) Mandataire: Godard, Xavier
(86) Numéro de dépôt international: PCT/FR2017/050085
(87) Numéro de publication internationale: WO 2017/125667

(56) Documents cités:
- WO-A1-2012/048050
- US-A1- 2003 220 641
- US-A1- 2007 093 840
- US-A1- 2010 191 248
- US-A1- 2012 203 231

## Description

La présente invention est relative à un dispositif de coupe flexible et canulé pour le perçage et/ou le fraisage d'un espace dans des tissus osseux ou conjonctifs.

On connait d'après le brevet antérieur US2013/0261628 du 7 janvier 2013 un foret souple de perçage comprenant une partie de tige proximale pour le raccordement à un dispositif d'entrainement en rotation, une partie distale constituée d'une pointe de coupe permettant de percer les tissus osseux; et une partie d'arbre intermédiaire s'étendant entre la partie de tige proximale et la partie distale. La partie d'arbre intermédiaire présente d'une part une souplesse longitudinale suffisante pour permettre au foret de se déformer selon un rayon de courbure, et d'autre part une résistance à la torsion suffisante pour permettre au foret le perçage d'un trou dans les tissus osseux.

On connait également d'après le brevet antérieur US6053922 du 17 juillet 1996 un arbre flexible utilisé pour le perçage d'un alésage dans les tissus osseux. L'arbre flexible est constitué d'un élément tubulaire présentant un alésage longitudinal interne sur toute la longueur dudit élément et une fente de forme appropriée qui se prolonge en spirale continue ou par segments continus autour dudit élément tubulaire. L'élément tubulaire comporte à chaque extrémité opposée de l'arbre d'une part une tête de coupe et d'autre part un moyen de connexion à un dispositif d'entraînement.

Le brevet antérieur US 2003/220641 A1 définit le préambule de la revendication 1.

On note que les dispositifs de perçage de l'art antérieur ne permettent pas de percer et de fraiser l'espace intervertébrale se trouvant entre deux vertèbres d'un segment rachidien en passant par les pédicules de ces dernières.

En effet, les dispositifs de perçage de l'art antérieur assurent uniquement le perçage des tissus osseux suivant une direction sensiblement courbe sans contrôle possible dudit rayon de courbure et sans garantie du point de perçage.

Le dispositif de coupe flexible et canulé pour le perçage et/ou le fraisage d'un espace dans des tissus osseux ou conjonctifs suivant la présente invention comporte d'une part un élément de coupe constitué d'un tube rigide solidaire à l'une de ses extrémités d'une liaison souple et flexible se prolongeant par une fraise canulée et à l'extrémité opposée de moyens d'entrainement pourvus d'un connecteur de sécurité pour le désaccouplement dudit dispositif de coupe et d'autre part un élément de guidage assurant le positionnement et la déformation angulaire de l'élément de coupe à l'intérieur des tissus osseux ou conjonctifs.

Le dispositif de coupe flexible et canulé suivant la présente invention comporte un élément de coupe dont la liaison souple et flexible est constituée d'un toron creux à fibres ou fils hélicoïdaux autour duquel est disposé une gaine de protection qui sont respectivement assemblés à la fraise canulée d'une part et au tube rigide d'autre part.

Le dispositif de coupe flexible et canulé suivant la présente invention comprend un toron creux qui est constitué d'un agencement d'au moins trois couches de fibres ou fils hélicoïdaux disposées en quinconce délimitant un alésage interne.

Le dispositif de coupe flexible et canulé suivant la présente invention comporte un toron creux dont chaque couche est formée d'au moins dix huit fibres ou fils agencés régulièrement sur la périphérie circulaire dudit toron selon un profil en hélice suivant l'axe longitudinal dudit toron.

Le dispositif de coupe flexible et canulé suivant la présente invention comporte un toron creux dont la première et la troisième couche sont disposées suivant l'axe longitudinal dudit toron selon une même première direction en hélice, tandis que la seconde couche intercalée entre la première et la troisième est dirigée suivant une seconde direction en hélice qui coupe ladite première direction en hélice.

Le dispositif de coupe flexible et canulé suivant la présente invention comprend un toron creux qui est fixé à l'une de ses extrémités sur le bord vertical d'une première partie cylindrique d'une entretoise tubulaire solidaire du tube rigide tandis que l'autre extrémité est assemblée sur le bord vertical d'un épaulement de la fraise canulée.

Le dispositif de coupe flexible et canulé suivant la présente invention comprend une liaison souple et flexible dont la gaine de protection est constituée d'un ressort à spires jointives venant envelopper la face externe des fibres ou fils hélicoïdaux du toron creux et dont chaque extrémité est respectivement solidaire du tube rigide et de la fraise canulée.

Le dispositif de coupe flexible et canulé suivant la présente invention comprend une liaison souple et flexible dont la gaine de protection est formée d'une gaine en polymère souple dont chaque extrémité est fixée au tube rigide et à la fraise canulée.

Le dispositif de coupe flexible et canulé suivant la présente invention comporte un élément de coupe flexible dont la fraise canulée comporte au moins trois dents régulièrement réparties autour d'un alésage interne et dont le profil de coupe assure respectivement un avalement de matière provoquant lors de l'entrainement en rotation dudit dispositif de coupe son avancement dans les tissus osseux ou conjonctifs.

Le dispositif de coupe flexible et canulé suivant la présente invention comporte un élément de coupe flexible dont le connecteur de sécurité est solidaire du tube rigide par l'intermédiaire d'une goupille tarée pour rompre sous une couple qui est inférieure à celui de la liaison souple et flexible avec ledit tube rigide.

Le dispositif de coupe flexible et canulé suivant la présente invention comporte un élément de guidage qui est constitué d'une tige réalisée en Nitinol présentant un faible diamètre et une extrémité à profil courbe de rayon prédéterminé se terminant par une pointe effilée.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue en coupe illustrant un segment rachidien dont les tissus osseux ou conjonctif sont percés et/ou fraisés par le dispositif de coupe flexible et canulé suivant la présente invention.

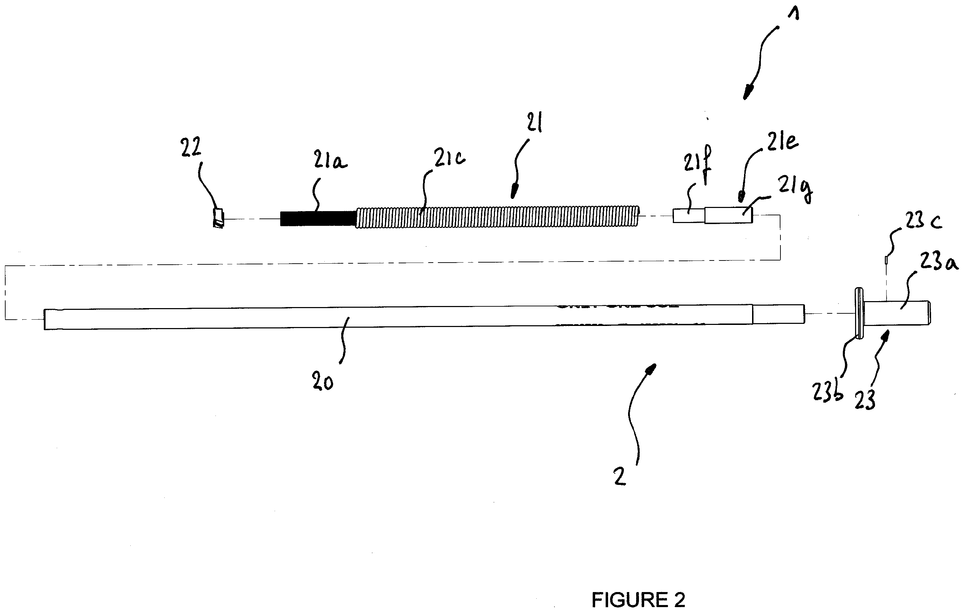

Figure 2 est une vue en perspective éclatée montrant les différentes pièces constituant l'élément de coupe du dispositif de coupe flexible et canulé suivant la présente invention.

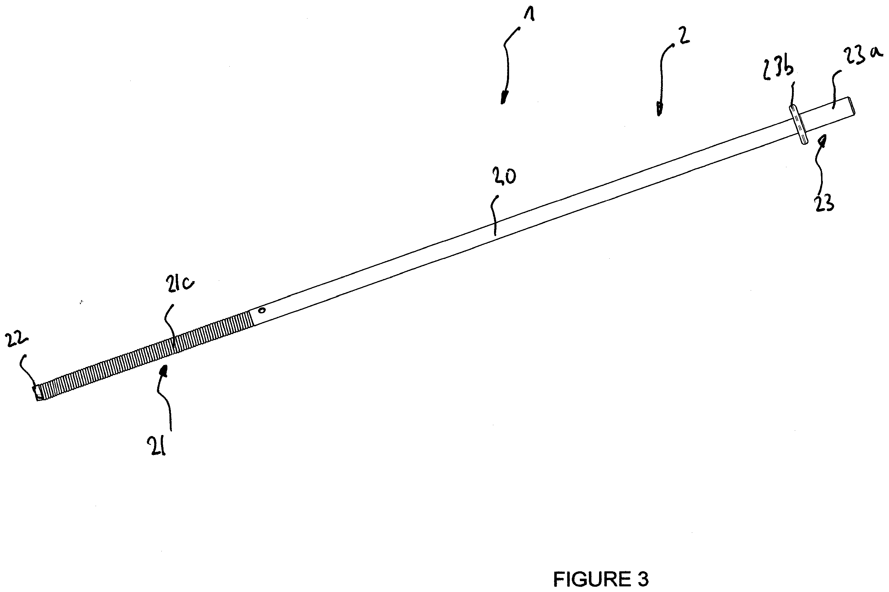

Figure 3 est une vue en perspective représentant l'élément de coupe assemblé du dispositif de coupe flexible et canulé suivant la présente invention.

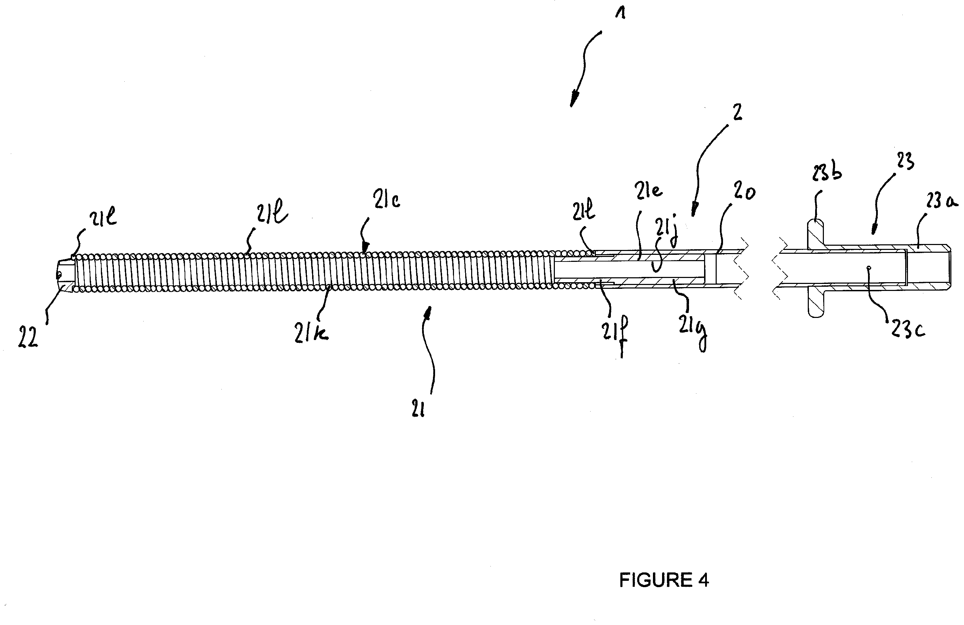

Figure 4 est une vue en coupe illustrant en détail l'agencement de la liaison souple de l'élément de coupe du dispositif de coupe flexible et canulé suivant la présente invention.

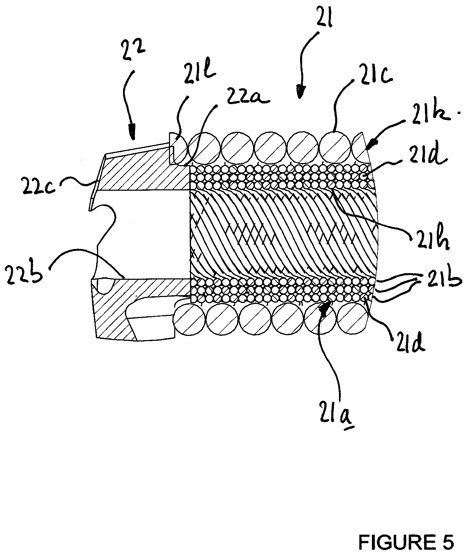

Figure 5 est une vue en coupe représentant la fixation de la liaison souple de l'élément de coupe au niveau de la fraise canulée du dispositif de coupe flexible et canulé suivant la présente invention.

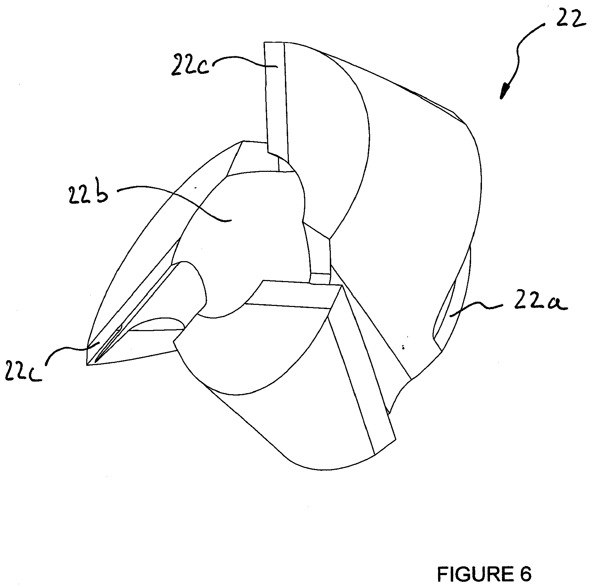

Figure 6 est une vue en perspective montrant le profil de la fraise canulée du dispositif de coupe flexible et canulé suivant la présente invention.

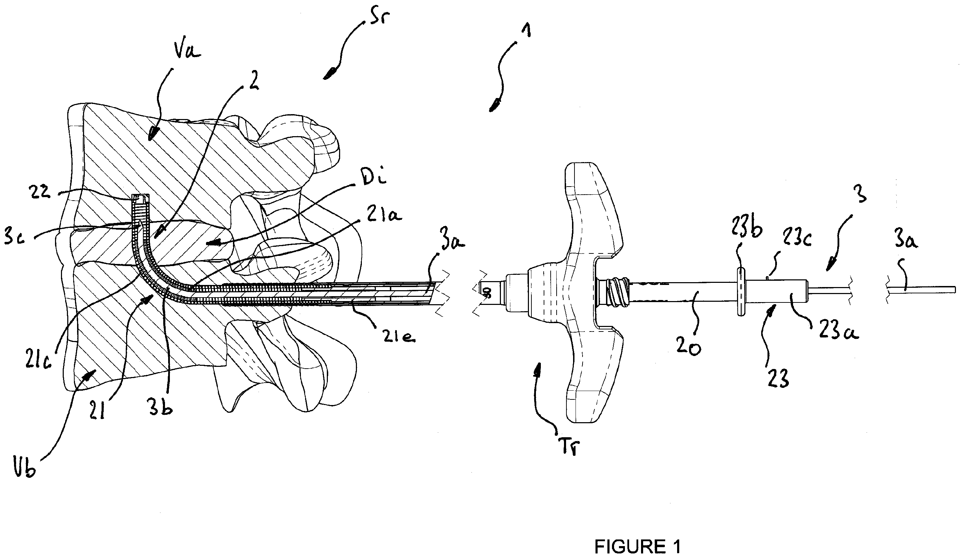

On a montré en figure 1 un segment rachidien Sr d'une colonne vertébral dont les vertèbres Va, Vb et le disque intervertébral Di sont percés et/ou fraisés par un dispositif de coupe flexible et canulé 1 suivant la présente invention.

Préalablement à la mise en place du dispositif de coupe 1, il est ancré par exemple dans la vertèbre inférieure Vb un trocart droit Tr permettant le guidage dudit dispositif de coupe 1 dans la partie de perçage horizontale et droite de ladite vertèbre Va.

Le dispositif de coupe 1 est constitué d'un élément de coupe flexible et canulé 2 et d'un élément de guidage 3 à profil courbe assurant le positionnement et la déformation angulaire de l'élément de coupe 2 à l'intérieur des tissus osseux et/ou conjonctifs.

L'élément de guidage 3 du dispositif de coupe 1 est constitué d'une tige 3a réalisée en Nitinol présentant un faible diamètre et une extrémité à profil courbe 3b de rayon prédéterminé se terminant par une pointe effilé 3c.

L'élément de guidage 3 est réalisé dans un matériau hyper élastique assurant une déformation élastique de l'extrémité à profil courbe 3b afin de permettre son introduction dans le trocart droit Tr.

L'élément de coupe flexible et canulé 2 du dispositif de coupe 1 est constitué d'un tube rigide 20 solidaire à l'une de ses extrémités d'une liaison souple et flexible 21 se prolongeant par une fraise canulée 22 et à l'extrémité opposée d'un connecteur de sécurité 23 permettant la liaison avec des moyens d'entrainement non représentés.

La liaison souple et flexible 21 est constituée d'un toron creux 21a à fibres ou fils hélicoïdaux 21d autour duquel est disposé une gaine de protection 21c qui sont respectivement assemblés à la fraise canulée 22 d'une part et au tube rigide 20 d'autre part.

Le toron creux 21a est constitué d'un agencement d'au moins trois couches 21b de fibres ou fils hélicoïdaux 21d disposées en quinconce délimitant un alésage interne 21h.

Chaque couche 21b est formée d'au moins dix huit fibres ou fils 21d agencés régulièrement sur la périphérie circulaire du toron 21a selon un profil en hélice suivant l'axe longitudinal dudit toron.

La première et la troisième couche 21b sont disposées suivant l'axe longitudinal du toron 21a selon une même première direction en hélice, tandis que la seconde couche 21b intercalée entre la première et la troisième est dirigée suivant une seconde direction en hélice qui coupe ladite première direction en hélice.

Chaque fibre ou fils 21d de chaque couche 21b du toron creux 21a présente un même diamètre externe.

Le toron creux 21a coopère à l'une de ses extrémités avec le bord vertical d'une première partie cylindrique 21f d'une entretoise tubulaire 21e sur laquelle sont soudées les extrémités libres des fibres ou fils hélicoïdaux 21d des trois couches 21b disposées en quinconce.

Le toron creux 21a est positionné dans le prolongement de la première partie cylindrique 21f de l'entretoise 21e afin que les alésages internes 21h et 21j soient respectivement coaxiaux.

L'entretoise 21e comporte dans le prolongement de la première partie cylindrique 21f une seconde partie cylindrique 21g dont le diamètre externe est supérieur à celui de ladite première afin de coopérer avec l'alésage interne du tube rigide 20.

La seconde partie cylindrique 21g de l'entretoise 21e est immobilisée en translation et en rotation à l'intérieur du tube rigide 20 par tout moyen de fixation comme par exemple par soudage et/ou par déformation axiale de la parois externe dudit tube.

Les autres extrémités libres des fibres ou fils hélicoïdaux 21d des trois couches 21b disposées en quinconce du toron creux 21a opposées à celles solidaires de l'entretoise 21e sont fixées par soudage sur le bord vertical d'un épaulement 22a de la fraise canulée 22.

Le toron creux 21a est positionné par rapport à la fraise canulée 22 de manière que les alésages internes 21h et 22b soient respectivement dans le prolongement de l'un de l'autre.

La gaine de protection 21c de la liaison souple et flexible 21 est constituée d'un ressort 21k à spires jointives 21l venant envelopper la face externe des fibres ou fils hélicoïdaux 21d du toron creux 21a et dont chaque extrémité est respectivement solidaire du tube rigide 20 et de la fraise canulée 22.

Dans cette exemple de réalisation la première extrémité libre du ressort 21k est solidaire du tube rigide 20 par le soudage de la première spire 21l sur le bord vertical de l'extrémité libre dudit tube.

La seconde extrémité libre du ressort 21k est solidaire de la fraise canulée 22 par le soudage de la dernière spire 21l sur la périphérie externe de l'épaulement 22a à proximité de la zone de solidarisation du toron creux 21 sur ce même épaulement.

Dans une variante de réalisation la gaine de protection 21c peut être constituée d'une gaine en polymère souple dont chaque extrémité peut être fixée directement ou indirectement sur le tube rigide 20 et la fraise canulée 22.

La fraise canulée 22 de l'élément de coupe flexible 2 présente au moins trois dents 22c régulièrement réparties autour de l'alésage interne 22b et dont le profil de coupe assure respectivement un avalement de matière provoquant lors de l'entrainement en rotation du dispositif de coupe 1 son avancement dans les tissus osseux ou conjonctifs.

La fraise canulée 22 et avaleuse permet de diminuer les contraintes de compression transmise à la liaison souple et flexible 21 lors des forages ou fraisages. Les angles de coupe des dents 22c sont adaptés afin de réduire les efforts axiaux de compression lors de l'enlèvement de tissus osseux ou conjonctifs.

Le connecteur de sécurité 23 de l'élément de coupe flexible et canulé 2 présente un couple de rupture qui est inférieur à celui de la liaison souple et flexible 21 avec le tube rigide 20 assurant un désaccouplement dudit dispositif de coupe 1 avec les moyens d'entrainement lorsque la fraise canulée 22 rencontre un tissu dense entrainant une augmentation du couple de résistance.

Le connecteur de sécurité 23 est constitué d'un manchon cylindrique 23a solidaire à l'une de ses extrémités d'une collerette 23b. Le manchon 23a présente un diamètre interne permettant de recevoir celui externe du tube rigide 20.

Le connecteur de sécurité 23 est rendu solidaire du tube rigide 20 par l'intermédiaire d'une goupille 23c traversant le manchon 23a et ledit tube. La goupille 23c est tarée pour rompre sous un couple qui est inférieure à celui de la liaison souple et flexible 21 avec le tube rigide 20.

On comprend que pour éviter la rupture de la liaison souple et flexible 21 dans le corps du patient, la rupture est limitée par un couple maximal que peut supporter la goupille 23c qui est dimensionnée afin de rompre avant ladite liaison souple et flexible 21.

L'agencement structurel de l'élément de coupe flexible et canulé 2 et la manière dont sont fixées les extrémités de la liaison souple et flexible 21 au tube rigide 20 et à la fraise canulée 22 permet d'une part que le toron creux 21a transmette le couple même le long de l'élément de guidage courbe 3 et d'autre part que la gaine de protection 21c subisse les efforts axiaux et radiaux sans rupture.

Il doit d'ailleurs être entendu que la description qui précède n'a été donnée qu'à titre d'exemple, et quelle ne limite nullement le domaine de l'invention dont l'étendue n'est limitée que par les revendications.

## Revendications

1. Dispositif de coupe flexible et canulé pour le perçage et/ou le fraisage d'un espace dans des tissus osseux ou conjonctifs, ledit dispositif comportant d'une part un élément de coupe (2) constitué d'un tube rigide (20) solidaire à l'une de ses extrémités d'une liaison souple et flexible (21) se prolongeant par une fraise canulée (22) et à l'extrémité opposée de moyens d'entrainement pourvus d'un connecteur de sécurité (23) pour le désaccouplement dudit dispositif de coupe et d'autre part d'un élément de guidage (3) assurant le positionnement et la déformation angulaire de l'élément de coupe (2) à l'intérieur des tissus osseux ou conjonctifs, **caractérisé en ce que** ladite liaison souple et flexible (21) de l'élément de coupe (2) est constituée d'un toron creux (21a) à fibres ou fils hélicoïdaux (21d) autour duquel est disposée une gaine de protection (21c), ledit toron (21a) et ladite gaine de protection (21c) étant respectivement assemblés à ladite fraise canulée (22) et au tube rigide (20).

2. Dispositif de coupe flexible et canulé suivant la revendication 2, **caractérisé en ce que** le toron creux (21a) est constitué d'un agencement d'au moins trois couches (21b) de fibres ou fils hélicoïdaux (21d) disposées en quinconce délimitant un alésage interne (21h).

3. Dispositif de coupe flexible et canulé suivant la revendication 3, **caractérisé en ce que** chaque couche (21b) est formée d'au moins dix huit fibres ou fils (21d) agencés régulièrement sur la périphérie circulaire du toron (21a) selon un profil en hélice suivant l'axe longitudinal dudit toron.

4. Dispositif de coupe flexible et canulé suivant la revendication 3, **caractérisé en ce que** la première et la troisième couche (21b) sont disposées suivant l'axe longitudinal du toron (21a) selon une même première direction en hélice, tandis que la seconde couche (21b) intercalée entre la première et la troisième est dirigée suivant une seconde direction en hélice qui coupe ladite première direction en hélice.

5. Dispositif de coupe flexible et canulé suivant la revendication 3, **caractérisé en ce que** le toron creux (21a) est fixé à l'une de ses extrémités sur le bord vertical d'une première partie cylindrique (21f) d'une entretoise tubulaire (21e) solidaire du tube rigide (20) tandis que l'autre extrémité est assemblée sur le bord vertical d'un épaulement (22a) de la fraise canulée (22).

6. Dispositif de coupe flexible et canulé suivant la revendication 2, **caractérisé en ce que** la gaine de protection (21c) de la liaison souple et flexible (21) est constituée d'un ressort (21k) à spires jointives (21l) venant envelopper la face externe des fibres ou fils hélicoïdaux (21d) du toron creux (21a) et dont chaque extrémité est respectivement solidaire du tube rigide (20) et de la fraise canulée (22).

7. Dispositif de coupe flexible et canulé suivant la revendication 2, **caractérisé en ce que** la gaine de protection (21c) peut être constituée d'une gaine en polymère souple dont chaque extrémité peut être fixée directement ou indirectement sur le tube rigide (20 et la fraise canulée (22).

8. Dispositif de coupe flexible et canulé suivant la revendication 1, **caractérisé en ce que** la fraise canulée (22) de l'élément de coupe flexible (2) présente au moins trois dents (22c) régulièrement réparties autour d'un alésage interne (22b) et dont le profil de coupe assure respectivement un avalement de matière provoquant lors de l'entrainement en rotation dudit dispositif de coupe (1) son avancement dans les tissus osseux ou conjonctifs.

9. Dispositif de coupe flexible et canulé suivant la revendication 1, **caractérisé en ce que** le connecteur de sécurité (23) est solidaire du tube rigide (20) par l'intermédiaire d'une goupille (23c) tarée pour rompre sous un couple qui est inférieure à celui de la liaison souple et flexible (21) avec ledit tube rigide (20).

10. Dispositif de coupe flexible et canulé suivant la revendication 1, **caractérisé en ce que** l'élément de guidage (3) est constitué d'une tige (3a) réalisé en Nitinol présentant un faible diamètre et une extrémité à profil courbe (3b) de rayon prédéterminé se terminant par une pointe effilée (3c).

## Patentansprüche

1. Flexible und kanülierte Schneidvorrichtung zum Bohren und/oder Fräsen eines Raums in Knochen- oder Bindegeweben, wobei die Vorrichtung
einerseits ein Schneidelement (2), das aus einem starren Rohr (20) besteht, das an einem seiner Enden mit einer biegsamen und flexiblen Verbindung (21), die sich durch einen kanülierten Fräser (22) erstreckt, und am gegenüberliegenden Ende mit Antriebsmitteln fest verbunden ist, die mit einem Sicherheitsverbinder (23) zum Lösen der Schneidvorrichtung versehen sind,
und andererseits ein Führungselement (3) aufweist, das das Positionieren und die Winkelverformung des Schneidelements (2) im Inneren der Knochen- oder Bindegewebe sicherstellt, **dadurch gekennzeichnet, dass** die biegsame und flexible Verbindung (21) des Schneidelements (2) aus einem Hohlstrang (21a) mit Fasern oder schraubenförmig gewickelten Drähten (21d) besteht, um den eine Schutzhülle (21c) angeordnet ist, wobei der Strang (21a) und die Schutzhülle (21c) jeweils an dem kanülierten Fräser (22) und an dem starren Rohr (20) angebracht sind.

2. Flexible und kanülierte Schneidvorrichtung nach Anspruch 2, **dadurch gekennzeichnet, dass** der Hohlstrang (21a) aus einer Anordnung von mindestens drei Schichten (21b) von Fasern oder schraubenförmig gewickelten Drähten (21d) gebildet ist, die versetzt angeordnet sind, die eine innere Bohrung (21h) begrenzen.

3. Flexible und kanülierte Schneidvorrichtung nach Anspruch 3, **dadurch gekennzeichnet, dass** jede Schicht (21b) aus mindestens achtzehn Fasern oder Drähten (21d) gebildet ist, die regelmäßig am kreisförmigen Umfang des Strangs (21a) in einem spiralförmigen Profil entlang der Längsachse des Strangs angeordnet sind.

4. Flexible und kanülierte Schneidvorrichtung nach Anspruch 3, **dadurch gekennzeichnet, dass** die erste und die dritte Schicht (21b) entlang der Längsachse des Strangs (21a) in der gleichen ersten spiralförmigen Richtung angeordnet sind, während die zweite Schicht (21b), die zwischen der ersten und der dritten angeordnet ist, in eine zweite spiralförmige Richtung gerichtet ist, die die erste spiralförmige Richtung schneidet.

5. Flexible und kanülierte Schneidvorrichtung nach Anspruch 3, **dadurch gekennzeichnet, dass** der Hohlstrang (21a) an einem seiner Enden an der vertikalen Kante eines ersten zylindrischen Teils (21f) eines röhrenförmigen Abstandshalters (21e) befestigt ist, der mit dem starren Rohr (20) fest verbunden ist, während das andere Ende an der vertikalen Kante einer Schulter (22a) des kanülierten Fräsers (22) angebracht ist.

6. Flexible und kanülierte Schneidvorrichtung nach Anspruch 2, **dadurch gekennzeichnet, dass** die Schutzhülle (21c) der biegsamen und flexiblen Verbindung (21) aus einer Feder (21k) mit aneinander liegenden Windungen (211) besteht, die die Außenseite der Fasern oder schraubenförmig gewickelten Drähte (21d) des Hohlstrangs (21a) umhüllen und wovon jedes Ende jeweils mit dem starren Rohr (20) und des kanülierten Fräsers (22) fest verbunden ist.

7. Flexible und kanülierte Schneidvorrichtung nach Anspruch 2, **dadurch gekennzeichnet, dass** die Schutzhülle (21c) aus einer flexiblen Polymerhülle bestehen kann, wovon jedes Ende direkt oder indirekt an dem starren Rohr (20) und dem kanülierten Fräser (22) befestigt werden kann.

8. Flexible und kanülierte Schneidvorrichtung nach Anspruch 1, **dadurch gekennzeichnet, dass** der kanülierte Fräser (22) des flexiblen Schneidelements (2) mindestens drei Zähne (22c) aufweist, die regelmäßig um eine Innenbohrung (22b) verteilt sind und deren Schnittprofil jeweils ein Einziehen von Material gewährleistet, wodurch während des Indrehungversetzens der Schneidvorrichtung (1) ihr Vorschub in die Knochen- oder Bindegewebe bewirkt wird.

9. Flexible und kanülierte Schneidvorrichtung nach Anspruch 1, **dadurch gekennzeichnet, dass** der Sicherheitsverbinder (23) mit dem starren Rohr (20) über einen Stift (23c) fest verbunden ist, der kalibriert ist, um bei einem Drehmoment zu brechen, das niedriger ist als jenes der biegsamen und flexiblen Verbindung (21) mit dem starren Rohr (20).

10. Flexible und kanülierte Schneidvorrichtung nach Anspruch 1, **dadurch gekennzeichnet, dass** das Führungselement (3) aus einer Stange (3a) besteht, die aus Nitinol hergestellt ist, die einen kleinen Durchmesser und ein Ende mit einem gekrümmten Profil (3b) mit einem vorbestimmten Radius aufweist, die in einer sich verjüngenden Spitze (3c) endet.

## Claims

1. Flexible and cannulated cutting device for drilling and/or milling a space in bone or connective tissue, said device comprising
on the one hand a cutting element (2) made up of a rigid tube (20) secured at one of its ends to a supple and flexible connection (21) extended by a cannulated bur (22) and at the opposite end to drive means provided with a safety connector (23) for uncoupling said cutting device
and on the other hand a guide element (3) providing the positioning and angular deformation of the cutting element (2) within the bone or connective tissues, **characterized in that** the said supple and flexible connection (21) of the cutting element (2) is made up of a hollow strand (21a) with helicoidal fibers or threads (21d) around which a protective sheath (21c) is arranged, said strand (21a) and said protective sheath (21c) being respectively assembled to said cannulated bur (22) and to the rigid tube (20).

2. Flexible and cannulated cutting device according to claim 2, **characterized in that** the hollow strand (21 a) is made up of an arrangement of at least three layers (21b) of helicoidal fibers or threads (21d) arranged in a staggered configuration delimiting an internal bore (21h).

3. Flexible and cannulated cutting device according to claim 3, **characterized in that** each layer (21b) is formed of at least eighteen fibers or threads (21d) arranged evenly at the circular periphery of the strand (21a) in a helical profile along the longitudinal axis of said strand.

4. Flexible and cannulated cutting device according to claim 3, **characterized in that** the first and the third layer (21b) are arranged along the longitudinal axis of the strand (21a) in one and the same first helical direction, whereas the second layer (21b) interposed between the first and the third is directed in a second helical direction that intersects said first helical direction.

5. Flexible and cannulated cutting device according to claim 3, **characterized in that** the hollow strand (21a) is fixed at one of its ends to the vertical edge of a first cylindrical part (21f) of a spacer tubular (21e) secured to the rigid tube (20) whereas the other end is assembled with the vertical edge of a shoulder (22a) of the cannulated bur (22).

6. Flexible and cannulated cutting device according to claim 2, **characterized in that** the protective sheath (21c) of the supple and flexible connection (21) is made up of a spring (21k) with contiguous turns (21l) enveloping the outer face of the helicoidal fibers or threads (21d) of the hollow strand (21a), each end of which being respectively secured to the rigid tube (20) and to the cannulated bur (22).

7. Flexible and cannulated cutting device according to claim 2, **characterized in that** the protective sheath (21c) may be made up of a flexible polymer sheath, each end of which may be fixed directly or indirectly on the rigid tube (20) and the cannulated bur (22).

8. Flexible and cannulated cutting device according to claim 1, **characterized in that** the cannulated bur (22) of the flexible cutting element (2) has at least three teeth (22c) evenly distributed around an internal bore (22b) and which cutting profile respectively provides a removal of waste material causing, when said cutting device (1) is rotationally driven, the advance thereof into the bone or connective tissues.

9. Flexible and cannulated cutting device according to claim 1, **characterized in that** the safety connector (23) is secured to the rigid tube (20) via a pin (23c) so designed as to break under a torque which is lower than that of the supple and flexible connection (21) connected to said rigid tube (20).

10. Flexible and cannulated cutting device according to claim 1, **characterized in that** the guide element (3) is made up of a rod (3a) made of Nitinol having a small diameter and an end with a curved profile (3b) of predetermined radius ending in a tapered point (3c).
